# EUROPÄISCHE PATENTANMELDUNG

(11) **EP 1 283 206 A2**
(43) Veröffentlichungstag der Anmeldung: **12.02.2003**
(21) Anmeldenummer: 02016827.4
(22) Anmeldetag: 29.07.2002
(51) Int. Cl.: C07D 301/08, C07D 301/03

(54) **Verfahren zur Epoxidierung von Kohlenwasserstoffen**

(30) Priorität: 10.08.2001 DE 10139531
(71) Anmelder: BAYER AG, 51368 Leverkusen (DE)
(72) Erfinder: Jansen, Ursula, Dr., 41470 Neuss (DE); Wegner, Andreas, Dr., 45468 Mühlheim (DE)

(57) **Zusammenfassung**

Die vorliegende Erfindung betrifft ein Verfahren zur Epoxidierung von Kohlenwasserstoffen mit Sauerstoff oder Luft oder Stickstoffoxiden oder anderen gasförmigen Oxidationsmitteln, dadurch gekennzeichnet, dass das Verfahren in Gegenwart einer Mischung enthaltend wenigstens zwei Elemente aus der Gruppe bestehend aus Sc, Y, Ti, Zr, Hf, V, Nb, Ta, Cr, Mo, W, Re, Fe, Co, Ni, Sn, Pb, Sb, Bi und Se auf einem Träger mit einer BET-Oberfläche von weniger als 200 m²/g durchgeführt wird sowie die Verwendung einer Mischung enthaltend wenigstens zwei Metalle aus der Gruppe bestehend aus Sc, Y, Ti, Zr, Hf, V, Nb, Ta, Cr, Mo, W, Re, Fe, Co, Ni, Sn, Pb, Sb, Bi und Se auf einem Träger mit einer BET-Oberfläche von weniger als 200 m²/g zur Epoxidierung von Kohlenwasserstoffen.

## Beschreibung

Die vorliegende Erfindung betrifft ein Verfahren zur Epoxidierung von Kohlenwasserstoffen mit Sauerstoff, dadurch gekennzeichnet, dass das Verfahren in Gegenwart einer Mischung enthaltend wenigstens zwei Elemente aus der Gruppe bestehend aus Sc, Y, Ti, Zr, Hf, V, Nb, Ta, Cr, Mo, W, Re, Fe, Co, Ni, Sn, Pb, Sb, Bi und Se auf einem Träger mit einer BET-Oberfläche von weniger als 200 m²/g durchgeführt wird sowie die Verwendung einer Mischung enthaltend wenigstens zwei Elemente aus der Gruppe bestehend aus Sc, Y, Ti, Zr, Hf, V, Nb, Ta, Cr, Mo, W, Re, Fe, Co, Ni, Sn, Pb, Sb, Bi und Se auf einem Träger mit einer BET-Oberfläche von weniger als 200 m²/g zur Epoxidierung von Kohlenwasserstoffen.

Epoxide sind ein wichtiges Ausgangsmaterial für die Polyurethanindustrie. Für deren Herstellung gibt es eine Reihe von Verfahren, die zum Teil auch technisch umgesetzt wurden. Für die industrielle Herstellung von Ethylenoxid verwendet man heute die Direktoxidation von Ethen mit Luft bzw. mit molekularem Sauerstoff enthaltenden Gasen in Gegenwart eines silberhaltigen Katalysators, wie in EP-A2-933 130 beschrieben. Um längerkettige Epoxide herzustellen, werden in technischem Maßstab in der Regel Wasserstoffperoxid oder Hypochlorit als Oxidationsmittel in der Flüssigphase eingesetzt. EP-A1-0 930 308 beschreibt z.B. den Gebrauch von Ionenausgetauschten Titansilikaliten als Katalysator mit diesen beiden Oxidationsmitteln.

Eine weitere Klasse von Oxidationskatalysatoren, die es erlaubt Propen in der Gasphase zum entsprechenden Epoxid zu oxidieren, wurde in neuerer Zeit durch USA 5,623,090 bekannt. Hierbei wird Gold auf Anatas als Katalysator verwendet, als Oxidationsmittel dient Sauerstoff, der in Gegenwart von Wasserstoff eingesetzt wird. Das System zeichnet sich durch ein außergewöhnlich hohe Selektivität (S>95%) bzgl. der Propenoxidation aus. Nachteilig sind der geringe Umsatz und die Desaktivierung des Katalysators.

Über andere aktive Komponenten außer Silber und Gold zur selektiven Direktoxidation von Propen und höheren Alkenen in der Gasphase zu den Epoxiden ist in der Literatur nicht viel bekannt.

Da bisher keiner der veröffentlichten Katalysatoren befriedigende Ergebnisse bzgl. Aktivität und Selektivität der Direktoxidation von Propen zu Propenoxid zeigte, sollten alternativ zu den bekannten silber- und goldhaltigen Katalysatoren andere Wirkkomponenten untersucht werden. Wichtige Voraussetzung ist hierbei, dass die Oxidation nicht vollständig zur entsprechenden Säure oder zum Aldehyd, bzw. Keton erfolgt, sondern auf der Stufe des Epoxids endet.

Einige Mischungen der Elemente der Gruppen 3-10 bzw. 14 -16 des Periodensystems nach IUPAC 1986 sind in der Literatur bereits bekannt.

So werden Mischungen aus Eisen, Cobalt und Nickel auf unterschiedlichen Trägern zur Herstellung von Ammoniak eingesetzt. Exemplarisch für die sehr umfangreiche Literatur sei hier nur auf den Review von M. Appl verwiesen [Indian Chem.Eng., 1987, 7-29]. Weiterhin werden Mischungen aus Eisen und Cobalt auch bei der Oxidation von Cyclohexan zu Adipinsäure eingesetzt [US-A 5,547,905]. Die Bildung von Epoxiden wird nicht offenbart.

Überraschend hat sich nun gezeigt, dass sich mit Mischungen verschiedener Elemente Propenoxid durch Direktoxidation von Propen mit Sauerstoff oder Luft oder Stickstoffoxiden oder anderen gasförmigen Oxidationsmitteln herstellen lässt (Sauerstoff ist bevorzugt). Dies ist umso ungewöhnlicher, da die Oxidation auf der Stufe der Epoxide stehen bleibt, und nicht die entsprechenden Säuren, Ketone oder Aldehyde entstehen.

Gegenstand der Erfindung ist ein Verfahren zur Epoxidierung von Kohlenwasserstoffen mit Sauerstoff, dadurch gekennzeichnet, dass das Verfahren in Gegenwart einer Mischung enthaltend wenigstens zwei Elemente aus der Gruppe bestehend aus Sc, Y, Ti, Zr, Hf, V, Nb, Ta, Cr, Mo, W, Re, Fe, Co, Ni, Sn, Pb, Sb, Bi und Se auf einem inerten Träger mit einer BET-Oberfläche von weniger als 200 m²/g durchgeführt wird.

Die Elemente können dabei elementar oder in gebundener Form vorliegen.

Unter dem Begriff Kohlenwasserstoff werden ungesättigte oder gesättigte Kohlenwasserstoffe wie Olefine oder Alkane verstanden, die auch Heteroatome wie N, O, P, S oder Halogene enthalten können. Die zu oxidierende organische Komponente kann azyklisch, monozyklisch, bizyklisch oder polyzyklisch und kann monoolefinisch, diolefinisch oder polyolefinisch sein. Bei organischen Komponenten mit zwei oder mehreren Doppelbindungen können die Doppelbindungen konjugiert und nichtkonjugiert vorliegen. Bevorzugt werden Kohlenwasserstoffe oxidiert, aus denen solche Oxidationsprodukte gebildet werden, deren Partialdruck bei der Reaktionstemperatur niedrig genug liegt, um das Produkt ständig vom Katalysator zu entfernen.

Bevorzugt sind ungesättigte und gesättigte Kohlenwasserstoffe mit 2 bis 20, vorzugsweise 3 bis 10 Kohlenstoffatomen, insbesondere Propen, Propan, Isobutan, Isobutylen, 1-Buten, 2-Buten, cis-2-Buten, trans-2-Buten, 1,3-Butadien, Penten, Pentan, 1-Hexen, 1-Hexan, Hexadien, Cyclohexen, Benzol.

Der Sauerstoff kann in verschiedenster Form eingesetzt werden, wie molekularer Sauerstoff, Luft und Stickstoffoxid. Molekularer Sauerstoff wird bevorzugt.

Geeignete Mischungen sind bevorzugt binäre Mischungen der Elemente ausgewählt aus der Gruppe bestehend aus Sc, Y, Ti, Zr, Hf, V, Nb, Ta, Cr, Mo, W, Re, Fe, Co, Ni, Sn, Pb, Sb, Bi und Se.

Es kann vorteilhaft sein, der Elementmischung übliche Promotoren oder Moderatoren, wie Erdalkali- und/oder Alkaliionen als Hydroxide, Carbonate, Nitrate, Chloride eines oder mehrerer Erdalkali- und/oder Alkalimetalle und/oder Silber beizumischen. Diese sind in EP-A1-0 933 130 auf Seite 4, Zeile 39 ff beschrieben.

Die verschiedenen Elemente und Promotoren in den Mischungen können in einer Konzentration von 0 bis 100 Gew.-%, bezogen auf die gesamte Masse der aktiven Bestandteile, vorliegen. Bevorzugt sind 0,01 bis 99,99 Gew.-%, besonders bevorzugt sind 0,1 bis 99,9 Gew.-%. Die Summe der Anteile aller Elemente in der Mischung beträgt 100 Gew.-%. Der bevorzugte Bereich für die Promotoren beträgt 0,001 bis 35 Gew.-%, bezogen auf die gesamte Masse der aktiven Bestandteile.

Bevorzugt werden folgende Mischungen CoFe, CoRe, CoCr, CoNi, NiCr, Co-Fe, Co-Re, Co-Cr, Co-Ni, Ni-Cr, Sb-Fe, Co-Fe-Ag, Co-Pb-Ag, Ni-Pb-Ag, Ni-Co-Ag, Co-Fe-Sr-Ag, Co-Pb-Sr-Ag, Co-Pb-Fe-Ag, Co-Cs-Fe-Ag, Co-Cs-Pb-Ag, Co-Ba-Bi-Ag, Ni-Pb-Fe-Ag, Ni-Cs-Fe-Ag, Ni-Cs-Pb-Ag, Ni-Ba-Fe-Ag, Ni-Ba-Pb-Ag, Ni-Co-Sr-Ag, Ni-Co-Fe-Ag, Eu-Er-Pb-W, Mo-Pb-Sr-Ag, Fe-Pb-Sr-Ag, Fe-Pb-Sr-Re, Fe-Mo-Sr-Ag, Cr-Sr-Re-Ag, Cr-Fe-Re-Ag, Cr-Fe-Sr-Ag, Cr-Fe-Pb-Ag, Cr-Fe-Mo-Pb, Co-Fe-Sr-Ag, Co-Cr-Re-Ag, Co-Cr-Pb-Re, Co-Cr-Pb-Sr, Co-Cr-Mo-Ag, Co-Cr-Fe-Sr, Co-Cr-Fe-Pb, Co-Cr-Fe-Mo, Co-K-Pb-Ag, Co-Nd-Pb-Ag, Co-Fe-Pb-Ag, Co-Fe-K-Ag, Co-Cs-Pb-Ag, Co-Cs-Fe-Ag, SbFe.

Bei den Trägern handelt es sich um Verbindungen aus der Verbindungsklasse der Al₂O₃, SiO₂, CeO₂, TiO₂ mit BET-Oberflächen <200 m²/g, bevorzugt <100 m²/g, besonders bevorzugt <10 m²/g und ganz besonders bevorzugt <1 m²/g.

Die Porosität beträgt vorteilhaft 20 bis 60 % (Volumenteil des Trägers), insbesondere 30 bis 50 %.

Die Teilchengrösse der Träger richtet sich nach den Verfahrensbedingungen der Gasphasenoxidation und liegt üblicherweise im Bereich von 1/10 bis 1/20 des Reaktordurchmessers.

Die spezifische Oberfläche wird in üblicher Weise bestimmt nach Brunauer, Emmet und Teller, J. Am. Chem. Soc. 1938, 60, 309 (und nach DIN 66 131), die Porosität durch die Quecksilberporosimetrie und die Teilchengröße der Elementpartikel auf der Trägeroberfläche mittels Elektronenmikroskopie und Röntgendiffraktometrie.

Die Elementkonzentration auf dem Träger sollte in der Regel im Bereich von 0,001 bis 50 Gew.-%, bevorzugt 0,001 bis 20 Gew.-%, ganz besonders bevorzugt 0,01 bis 5 Gew.-% betragen (bezogen auf die gesamte Masse aus Träger und Elementen).

Die Erzeugung der Elementpartikel auf dem Träger ist nicht auf eine Methode beschränkt. Zur Generierung von Elementpartikeln seien hier einige Beispielverfahren wie Abscheidung-Ausfällung (Deposition-Precipitation) wie in EP-B-0 709 360 auf S. 3, Z. 38 ff. beschrieben, Imprägnierung in Lösung, Incipient-wetness, Kolloid-Verfahren, Sputtern, CVD (chemical vapor deposition), PVD (physical vapor deposition) genannt.

Unter Incipient-wetness wird die Zugabe einer Lösung enthaltend lösliche Elementverbindungen zum Trägermaterial verstanden, wobei das Volumen der Lösung auf dem Träger kleiner als oder gleich dem Porenvolumen des Trägers ist. Somit bleibt der Träger makroskopisch trocken. Als Lösungsmittel für Incipient Wetness können alle Lösungsmittel verwendet werden, in denen die Elementverläufer löslich sind, wie Wasser, Alkohole, (Kronen-)Ether, Ester, Ketone halogenierte Kohlenwasserstoffe usw.

Bevorzugt wird der Träger mit einer die Elementionen enthaltenden Lösung getränkt und anschließend getrocknet und reduziert. Weiterhin kann die Lösung zusätzlich dem Fachmann bekannte Komponenten enthalten, die die Löslichkeit des oder der Elementsalze im Lösungsmittel erhöhen können und/oder die die Redoxpotentiale der Elemente verändern und/oder den pH-Wert verändern. Insbesondere seien genannt Ammoniak, Amine, Diamine, Hydroxyamine und Säuren, wie HCl, HNO₃, H₂SO₄, H₃PO₄.
1. Das Tränken kann z.B. durch Incipient wetness geschehen, ist jedoch nicht auf dieses beschränkt. Der Incipient wetness-Prozess kann dabei folgende Schritte enthalten:
   - einmalige Belegung mit einem Element und/oder mehrmalige Belegung mit einem anderen Element
   - einmalige Belegung mit einem Teil der Elemente oder mit allen Elementen in einem Schritt,
   - mehrfache Belegung mit mehreren Elementen in einem oder mehreren Schritten hintereinander
   - mehrfache Belegung mit mehreren Elementen wechselseitig in einem oder mehreren Schritten
2. Trocknen des nach 1. erhaltenen Trägers mit den Aktivkomponenten bei einer Temperatur von etwa 40 bis etwa 200°C bei Normaldruck oder auch reduziertem Druck. Bei Normaldruck kann man unter Luftatmosphäre oder auch unter Inertgasatmosphäre (z.B. Ar, N₂, He et al.) arbeiten. Die Zeit der Trocknung liegt im Bereich von 2-24 h, bevorzugt von 4-8 h.
3. Calcinieren der nach 2 gewonnenen Katalysatorvorstufen unter Inertgasatmosphäre und anschließend/oder ausschließlich unter Sauerstoff enthaltender Gasatmosphäre. Die Gehalte an Sauerstoff im Gasstrom liegen vorteilhaft im Bereich von 0 bis 21 Vol.-%, bevorzugt von 5 bis 15 Vol.-% (bezogen auf das Volumen des gesamten Gasstromes). Die Temperatur für die Calcinierung wird dem Elementgemisch angepasst und liegt daher in der Regel im Bereich von 400 bis 600°C, bevorzugt bei 450 bis 550°C, besonders bevorzugt bei 500°C.
4. Reduzieren der nach 2 und/oder 3 gewonnenen Katalysatorvorstufen bei erhöhten Temperaturen unter Wasserstoff enthaltender Stickstoffatmosphäre. Der Gehalt an Wasserstoff kann zwischen 0 bis 100 Vol.-% liegen, bevorzugt jedoch bei 0 bis 25, besonders bevorzugt bei 5 Vol.-% (bezogen auf das Volumen des gesamten Gasstromes). Die Reduktionstemperaturen sind dem jeweiligen Elementgemisch angepasst und liegen zwischen 100 und 600°C.

Üblicherweise wird das Epoxidierungsverfahren, bevorzugt in der Gasphase, unter folgenden Bedingungen durchgeführt:

Die molare Menge des eingesetzten Kohlenwasserstoffs in Bezug auf die Gesamtmolzahl aus Kohlenwasserstoff, Sauerstoff und gegebenenfalls Verdünnungsgas sowie das relative molare Verhältnis der Komponenten kann in weiten Bereichen variiert werden und orientiert sich in der Regel an den Explosionsgrenzen des Kohlenwasserstoff-Sauerstoff-Gemisches. In der Regel wird oberhalb oder unterhalb der Explosionsgrenze gearbeitet.

Bevorzugt wird ein Überschuss von Kohlenwasserstoff, bezogen auf eingesetzten Sauerstoff (auf molarer Basis) eingesetzt. Der Kohlenwasserstoffgehalt im Sauerstoff ist typischerweise ≤ 2 Mol-% oder ≥ 78 Mol-% (bezogen auf die Summe aller Mole im Gasstrom). Bevorzugt werden Kohlenwasserstoffgehalte im Bereich von 0,5 bis 2 Mol-% bei Fahrweisen unterhalb der unteren Explosionsgrenze und 78 bis 99 Mol% bei Fahrweisen oberhalb der oberen Explosionsgrenze gewählt. Besonders bevorzugt sind jeweils die Bereiche von 1 bis 2 Mol-% bzw. 78 bis 90 Mol-%.

Der molare Sauerstoffanteil, in Bezug auf die Gesamtmolzahl aus Kohlenwasserstoff, Sauerstoff und Verdünnungsgas, kann in weiten Bereichen variiert werden. Bevorzugt wird der Sauerstoff im molaren Unterschuss zum Kohlenwasserstoff eingesetzt. Bevorzugt werden im Bereich von 1-21 Mol-%, besonders bevorzugt 5-21 Mol-% Sauerstoff eingesetzt (bezogen auf die gesamten Mole im Gasstrom).

Zu Kohlenwasserstoff und Sauerstoff kann optional auch ein Verdünnungsgas, wie Stickstoff, Helium, Argon, Methan, Kohlendioxid, Kohlenmonoxid oder ähnliche, sich überwiegend inert verhaltende Gase, eingesetzt werden. Auch Mischungen der beschriebenen Inertkomponenten können eingesetzt werden. Der Inertkomponentenzusatz ist zum Transport der freiwerdenden Wärme dieser exothermen Oxidationsreaktion und aus sicherheitstechnischen Gesichtspunkten günstig. In diesem Fall ist die oben beschriebene Zusammensetzung der Eduktgasmischungen auch in den Explosionsbereich der unverdünnten Mischung aus Kohlenwasserstoff und Sauerstoff hinein möglich.

Die Kontaktzeit von Kohlenwasserstoff und Katalysator beträgt in der Regel im Bereich von 5-60 Sekunden.

Der Prozess wird in der Regel bei Temperaturen im Bereich von 120-300°C, bevorzugt 180-250°C durchgeführt.

### Beispiele

### Beispiel 1

Man löst 2,468 g Cobaltnitrat in 3 ml Wasser, gibt die Lösung zu ca. 10 g Al₂O₃ und lässt diese aufsaugen. Man trocknet den so erhaltenen Feststoff 4 h bei 100°C in einem Vakuumtrockenschrank bei einem Vakuum von ca. 15 mm Hg.

Dann löst man 0,042 g Rheniumsäure in 4,5 ml Wasser, lässt diese Lösung vom vorher hergestellten Feststoff vollständig aufsaugen und trocknet über Nacht bei ca. 15 mm Hg und 100°C im Vakuumtrockenschrank.

Schließlich wird die so hergestellte Vorstufe 12 h bei 500°C mit 10 Vol.-% H₂ in N₂ mit 60 l/h reduziert.

Nach der Reduktion werden 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 230°C werden PO-Gehalte von ca. 60 ppm im Abgasstrom ermittelt.

### Beispiel 2

Man löst 2,468 g Cobaltnitrat in 3 ml Wasser und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 0,202 g Chromnitrat in 4,5 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 215°C werden PO-Gehalte von ca. 90 ppm im Abgasstrom ermittelt.

### Beispiel 3

Man löst 1,298 g Cobaltnitrat in 4 ml Wasser und läßt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 2,02 g Chromnitrat in 4 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 220°C werden PO-Gehalte von ca. 40 ppm im Abgasstrom ermittelt.

### Beispiel 4

Man löst 1,303 g Nickelnitrat in 4 ml Wasser und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 1,299 g Cobaltnitrat in 4,5 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 225°C werden PO-Gehalte von ca. 40 ppm im Abgasstrom ermittelt.

### Beispiel 5

Man löst 0,13 g Nickelnitrat in 4,5 ml Wasser und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 2,468 g Cobaltnitrat in 4 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 225°C werden PO-Gehalte von ca. 35 ppm im Abgasstrom ermittelt.

### Beispiel 6

Man löst 2,475 g Nickelnitrat in 3,0 ml Wasser und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 0,202 g Chromnitrat in 4,5 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 200°C werden PO-Gehalte von ca. 310 ppm im Abgasstrom ermittelt.

### Beispiel 7

Man löst 2,468 g Cobaltnitrat in 3 ml Wasser und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 0,19 g Eisennitrat in 4,5 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 225°C werden PO-Gehalte von ca. 45 ppm im Abgasstrom ermittelt.

### Beispiel 8

Man löst 0,129 g Cobaltnitrat in 4,5 ml Wasser und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 2,475 g Nickelnitrat in 3 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca, 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 215°C werden PO-Gehalte von ca. 15 ppm im Abgasstrom ermittelt.

### Beispiel 9

Man löst 0,646 g Antimonchlorid in 4,5 ml Ethanol und lässt diese Lösung von ca. 10 g Al₂O₃ aufsaugen. Man trocknet den so erhaltenen Feststoff 4 Stunden bei 100°C in einem Vakuumtrockenschrank bei einem Vakuum von ca. 15 mm Hg.

Dann löst man 1,9 g Eisennitrat in 3,5 ml Wasser, lässt diese Lösung vom vorher hergestellten Feststoff vollständig aufsaugen und trocknet über Nacht im Vakuumtrockenschrank bei ca. 15 mm Hg und 100°C.

Schließlich wird der Feststoff ca. 8 h bei 500°C mit 10 Vol.-% H₂ in N₂ mit 60 l/h reduziert.

Nach der Reduktion werden ca. 10 g des so erhaltenen Katalysators in einem kontinuierlich betriebenen Festbettreaktor bei einer Verweilzeit von ca. 20 sec unter einer Eduktgaszusammensetzung von 79 Vol.-% Propen und 21 Vol.-% Sauerstoff untersucht. Bei einer Innentemperatur von 225°C werden PO-Gehalte von ca. 30 ppm im Abgasstrom ermittelt.

### Beispiele 10 bis 50

### Präparation der Beispiele 10 - 50.

Die Katalysatoren, die in den Beispielen 10 bis 50 verwendet wurden, wurden nach der Incipient-wetness Methode hergestellt. Wässrige Lösungen der Elemente wurden hergestellt aus den Ausgangsmaterialien nach Tabelle 1. Die Lösungen enthielten 52,6 g des reinen Elements pro Liter Wasser für die Elemente, die in Tabelle 1 Vorgänger genannt werden. Die Lösungen enthielten 5,26 g des reinen Elements pro Liter Wasser für die Elemente, die in Tabelle 1 Promotor genannt werden.

**Tabelle 1**

| **Ausgangsmaterialien** | **Rolle** | **Element-Symbol** |
|---|---|---|
| Kobalt(II)nitrat | Vorgänger | Co |
| Chrom(III)nitrat | Vorgänger | Cr |
| Eisen(III)nitrat | Vorgänger | Fe |
| Ammoniumheptamolybdat* 4 H₂O | Vorgänger | Mo |
| Blei(II)nitrat | Vorgänger | Pb |
| Strontiumnitrat | Vorgänger | Sr |
| Rhenium(VII)oxid | Vorgänger | Re |
| Silbernitrat | Vorgänger | Ag |
| Caesiumnitrat | Vorgänger | Cs |
| Neodym(III)nitrat | Vorgänger | Nd |
| Kaliumnitrat | Vorgänger | K |
| Wismutnitrat | Vorgänger | Bi |
| Nickel(II)nitrat | Vorgänger | Ni |
| Bariumnitrat | Vorgänger | Ba |
| Europiumnitrat | Vorgänger | Eu |
| Erbium(III)nitrat | Vorgänger | Er |
| Natrium-meta-wolframat | Vorgänger | W |

Entsprechend der Anzahl an Elementen, die für einen bestimmten Katalysator erforderlich waren, wurden bis zu fünf wässrigen Lösungen der Elemente durch Mikro-Spritzen-Pumpen in ein 2 ml-Glasgefäß dosiert. Das gesamte Volumen der Lösungen in dem Glasgefäß betrug 450 Mikroliter. In Tabelle 2 bezieht sich "Zusammensetzung" auf den Anteil der entsprechenden wässrigen Element-Lösung an dem gesamten Volumen der Lösungen in dem Gefäß. Dann wurde 1 g Al₂O₃ der Lösung hinzugefügt. Nachdem die Lösung komplett durch das Al₂O₃ aufgesaugt worden war, hinzugefügt. Nachdem die Lösung komplett durch das Al₂O₃ aufgesaugt worden war, wurde das so erhaltene Material über Nacht bei ca. 100°C und 200 mbar in einem Vakuum-Trockenschrank getrocknet. Das Material wurde dann 4 Stunden bei 500°C in Luft kalziniert. Schließlich wurde der so erhaltene Katalysator in einen FestbettReaktor eingefüllt und für 4 Stunden bei 200°C konditioniert in einer Mischung aus Wasserstoff und Stickstoff (10 % Wasserstoff). Die Durchflussrate betrug dabei 0,08 Liter pro Stunde. Bei Normaldruck und 200°C wurde ein Gasstrom aus 24 % Propan, 4,5 % Sauerstoff und 71,5 % Luft mit einer Fließrate von 0,35 Liter pro Stunde über den Katalysator geleitet. Das ausströmende Gas wurde durch Gaschromatographie auf den Gehalt an Propylenoxid (PO) untersucht. In Tabelle 2 bedeutet "Ausbeute PO%" den Anteil an PO in Vol.-% an dem ausströmenden Gas.

**Tabelle 2**

| **Beispiel** | **Zusammensetzung** | **Ausbeute PO%** |
|---|---|---|
| 10 | Co0,3333Fe0,3333Ag0,3333 | 0,002804 |
| 11 | Co0,3333Pb0,3333Ag0,3333 | 0,012592 |
| 12 | Ni0,3333Pb0,3333Ag0,3333 | 0,006409 |
| 13 | Ni0,3333Co0,3333Ag0,3333 | 0,006942 |
| 14 | Co0,25Fe0,25Sr0,25Ag0,25 | 0,004742 |
| 15 | Co0,25Pb0,25Sr0,25Ag0,25 | 0,012488 |
| 16 | Co0,25Pb0,25Fe0,25Ag0,25 | 0,013624 |
| 17 | Co0,25Cs0,25Fe0,25Ag0,25 | 0,011009 |
| 18 | Co0,25Cs0,25Pb0,25Ag0,25 | 0,005510 |
| 19 | Co0,25Ba0,25Bi0,25Ag0,25 | 0,002667 |
| 20 | Ni0,25Pb0,25Fe0,25Ag0,25 | 0,006396 |
| 21 | Ni0,25Cs0,25Fe0,25Ag0,25 | 0,018402 |
| 22 | Ni0,25Cs0,25Pb0,25Ag0,25 | 0,002536 |
| 23 | Ni0,25Ba0,25Fe0,25Ag0,25 | 0,003577 |
| 24 | Ni0,25Ba0,25Pb0,25Ag0,25 | 0,003900 |
| 25 | Ni0,25Co0,25Sr0,25Ag0,25 | 0,002614 |
| 26 | Ni0,25Co0,25Fe0,25Ag0,25 | 0,005089 |
| 27 | Eu0,25Er0,25Pb0,25W0,25 | 0,008260 |
| 28 | Mo0,25Pb0,25Sr0,25Ag0,25 | 0,001332 |
| 29 | Fe0,25Pb0,25Sr0,25Ag0,25 | 0,001852 |
| 30 | Fe0,25Pb0,25Sr0,25Re0,25 | 0,002056 |
| 31 | Fe0,25Mo0,25Sr0,25Ag0,25 | 0,004513 |
| 32 | Cr0,25Sr0,25Re0,25Ag0,25 | 0,002423 |
| 33 | Cr0,25Fe0,25Re0,25Ag0,25 | 0,001870 |
| 34 | Cr0,25Fe0,25Sr0,25Ag0,25 | 0,002024 |
| 35 | Cr0,25Fe0,25Pb0,25Ag0,25 | 0,002467 |
| 36 | Cr0,25Fe0,25Mo0,25Pb0,25 | 0,003148 |
| 37 | Co0,25Fe0,25Mo0,25Pb0,25 | 0,001221 |
| 38 | Co0,25Cr0,25Re0,25Ag0,25 | 0,002484 |
| 39 | Co0,25Cr0,25Pb0,25Re0,25 | 0,001226 |
| 40 | Co0,25Cr0,25Pb0,25Sr0,25 | 0,001912 |
| 41 | Co0,25Cr0,25Mo0,25Ag0,25 | 0,001843 |
| 42 | Co0,25Cr0,25Fe0,25Sr0,25 | 0,002225 |
| 43 | Co0,25Cr0,25Fe0,25Pb0,25 | 0,001821 |
| 44 | Co0,25Cr0,25Fe0,25Mo0,25 | 0,002499 |
| 45 | Co0,25K0,25Pb0,25Ag0,25 | 0,006633 |
| 46 | Co0,25Nd0,25Pb0,25Ag0,25 | 0,003476 |
| 47 | Co0,25Fe0,25Pb0,25Ag0,25 | 0,005896 |
| 48 | Co0,25Fe0,25K0,25Ag0,25 | 0,008485 |
| 49 | Co0,25Cs0,25Pb0,25Ag0,25 | 0,006147 |
| 50 | Co0,25Cs0,25Fe0,25Ag0,25 | 0,008464 |

## Patentansprüche

1. Verfahren zur Epoxidierung von Kohlenwasserstoffen mit Sauerstoff oder Luft oder Stickstoffoxiden oder anderen gasförmigen Oxidationsmitteln, **dadurch gekennzeichnet, dass** das Verfahren in Gegenwart einer Mischung enthaltend wenigstens zwei Elemente aus der Gruppe bestehend aus Sc, Y, Ti, Zr, Hf, V, Nb, Ta, Cr, Mo, W, Re, Fe, Co, Ni, Sn, Pb, Sb, Bi und Se auf einem Träger mit einer BET-Oberfläche von weniger als 200 m²/g durchgeführt wird.

2. Verfahren nach Anspruch 1, **dadurch gekennzeichnet, dass** die BET-Oberfläche weniger als 100 m²/g beträgt.

3. Verfahren nach Anspruch 1 oder 2, dadurch charakterisiert, dass der Träger Al₂O₃ ist.

4. Verfahren nach einem der Ansprüche 1 bis 3, **dadurch gekennzeichnet, dass** der Kohlenwasserstoff ausgewählt wird aus der Gruppe Propen und Buten.

5. Verfahren nach einem der Ansprüche 1 bis 4, **dadurch gekennzeichnet, dass** eine oder mehrere der Elementmischungen CoFe, CoRe, CoCr, CoNi, NiCr, Co-Fe, Co-Re, Co-Cr, Co-Ni, Ni-Cr, Sb-Fe, Co-Fe-Ag, Co-Pb-Ag, Ni-Pb-Ag, Ni-Co-Ag, Co-Fe-Sr-Ag, Co-Pb-Sr-Ag, Co-Pb-Fe-Ag, Co-Cs-Fe-Ag, Co-Cs-Pb-Ag, Co-Ba-Bi-Ag, Ni-Pb-Fe-Ag, Ni-Cs-Fe-Ag, Ni-Cs-Pb-Ag, Ni-Ba-Fe-Ag, Ni-Ba-Pb-Ag, Ni-Co-Sr-Ag, Ni-Co-Fe-Ag, Eu-Er-Pb-W, Mo-Pb-Sr-Ag, Fe-Pb-Sr-Ag, Fe-Pb-Sr-Re, Fe-Mo-Sr-Ag, Cr-Sr-Re-Ag, Cr-Fe-Re-Ag, Cr-Fe-Sr-Ag, Cr-Fe-Pb-Ag, Cr-Fe-Mo-Pb, Co-Fe-Sr-Ag, Co-Cr-Re-Ag, Co-Cr-Pb-Re, Co-Cr-Pb-Sr, Co-Cr-Mo-Ag, Co-Cr-Fe-Sr, Co-Cr-Fe-Pb, Co-Cr-Fe-Mo, Co-K-Pb-Ag, Co-Nd-Pb-Ag, Co-Fe-Pb-Ag, Co-Fe-K-Ag, Co-Cs-Pb-Ag, Co-Cs-Fe-Ag, SbFe eingesetzt werden.

6. Katalysator enthaltend wenigstens zwei Elemente aus der Gruppe bestehend aus Sc, Y, Ti, Zr, Hf, V, Nb, Ta, Cr, Mo, W, Re, Fe, Co, Ni, Sn, Pb, Sb, Bi und Se auf einem Träger mit einer BET-Oberfläche von weniger als 200 m²/g.

7. Katalysator nach Anspruch 6, **dadurch gekennzeichnet, dass** die Elementmischung ausgewählt wird aus der Gruppe CoFe, CoRe, CoCr, CoNi, NiCr, SbFe.
